# EUROPÄISCHE PATENTSCHRIFT

(11) **EP 1 885 329 B1**
(45) Veröffentlichungstag und Bekanntmachung des Hinweises auf die Patenterteilung: **21.11.2012**
(21) Anmeldenummer: 06724756.9
(22) Anmeldetag: 09.05.2006
(51) Int. Cl.: A61K 8/60, A61K 8/81, A61Q 19/00, A61Q 19/08

(54) **KOSMETISCHE ZUBEREITUNGEN MIT EINEM GEHALT AN GLUCOSYLGLYCERIDEN UND EINEM ACRYLAMIDOMETHYLPROPYLSULFONSÄURE-VP-COPOLYMER**
COSMETIC PREPARATIONS CONTAINING GLUCOSYL GLYCERIDES AND AN ACRYLAMIDOMETHYL PROPYLSULPHONIC ACID - VP - COPOLYMER
PREPARATIONS COSMETIQUES CONTENANT DES GLUCOSYLGLYCERIDES ET UN COPOLYMERE D'ACIDE ACRYLAMIDOMETHYLPROPYLSULFONIQUE ET DE VP.

(30) Priorität: 19.05.2005 DE 102005023638
(43) Veröffentlichungstag der Anmeldung: 13.02.2008
(73) Patentinhaber: Beiersdorf AG, 20253 Hamburg (DE)
(72) Erfinder: BREITENBACH, Ute, 20251 Hamburg (DE); KALLMAYER, Volker, 22525 Hamburg (DE); RASCHKE, Thomas, 25421 Pinneberg (DE); SCHERNER, Cathrin, 22455 Hamburg (DE); SIEFKEN, Wilfried, 22529 Hamburg (DE); VIALA, Sophie, 20257 Hamburg (DE)
(86) Internationale Anmeldenummer: PCT/EP2006/004294
(87) Internationale Veröffentlichungsnummer: WO 2006/122669

(56) Entgegenhaltungen:
- EP-A- 0 770 378
- EP-A- 0 815 844
- EP-A- 0 824 915
- DE-A1- 10 065 047
- DATABASE WPI Section Ch, Week 200458 Derwent Publications Ltd., London, GB; Class B04, AN 2004-597337 XP002395733 & JP 2004 229668 A (TATSUMA HONKE SHUZO KK) 19. August 2004 (2004-08-19)
- R. Brummer and al.: "Rheological Swing Test to Predict the Temperature Stability of Cosmetic Emulsions" In: "XXIst IFSCC International Congress 2000, Berlin", pages 476-484,
- R. BRUNNER & AL.: "Zyklentest - eine rheologische Messtechnik zur Temperatur-Stabilitätsvorhersage von kosmetischen Emulsionen", SEIFEN, ÖLE UND WACHSE, no. 11, 2001, pages 38-40,

## Beschreibung

Die vorliegende Erfindung betrifft kosmetische Zubereitungen mit einem Gehalt an Glucosylglyceriden und einem oder mehreren Acrylamidomethylpropylsulfonsäure-Polymeren sowie deren Verwendung auf dem Gebiete der kosmetischen sowie der pharmazeutischen Dermatologie.

Die äußerste Schicht der Epidermis, das Stratum corneum (Homschicht), ist als wichtige Barriereschicht von besonderer Bedeutung u.a. für den Schutz vor Umwelteinflüssen und Austrocknung. Die Hornschicht wird im Kontakt mit der Umweit ständig abgenutzt und muß deshalb ununterbrochen erneuert werden.

Ein heute in der Fachwelt weitverbreitetes Hautmodell faßt das Stratum corneum als ZweiKomponenten-System, ähnlich einer Ziegelsteinmauer (Ziegelstein-Mörtel-Modell), auf. In diesem Modell entsprechen die Korneozyten (Homzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen. Hierzu zählen u.a. Polyole wie Glycerin, Sorbit und Xylit, ethoxylierte Polyole sowie hydrolysierte Proteine. Weitere Anwendung finden die im natürlichen Feuchthaltefaktor (sogenannter Natural Moisturizing Factor = NMF) enthaltenen Substanzen, wie z.B. Harnstoff, Kohlenhydrate (z. B. Glucose) und Aminosäuren (z. B. Serin). Diese Substanzen sind daher für die Pflegeleistung eines kosmetischen Produktes von besonderer Bedeutung, insbesondere auch aufgrund ihrer relativ guten Haut- und Schleimhautverträglichkeit.

Ungelöst ist jedoch das Problem, daß auch die grundsätzlich völlig unbedenklichen Substanzen Glucose und Glycerin in sehr hoher Dosierung bei besonders empfindlichen Personen bestimmte Haut- und Schleimhautreizungserscheinungen hervorrufen können. Ziel war es daher, feuchtigkeitsspendende Substanzen (sogenannte "Moisturizer") zu finden, die über eine noch bessere Verträglichkeit als beispielsweise Glucose und Glycerin verfügen.

Glucosylglyceride sind an sich vorzügliche Feuchtigkeitsspender für kosmetische Zwecke. Es hat sich aber herausgestellt, daß kosmetische Zubereitungen mit einem Gehalt an Glucosylglyceriden, zumal wenn sie einen Gehalt an herkömmlichen Verdickem aufweisen, sich nur durch unbefriedigende Stabilität auszeichnen.

Es war nach all diesem überraschend und nicht vorhersehbar, daß kosmetische,

Zubereitungen, enthaltend
(i) ein oder mehrere Glucosylglyceride gewählt aus den Substanzen der allgemeinen Formel 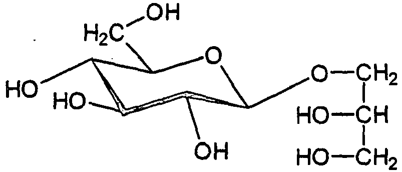 und/oder der allgemeinen Formel 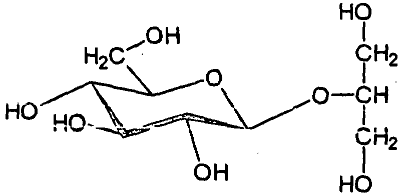 und/oder der allgemeinen Formel 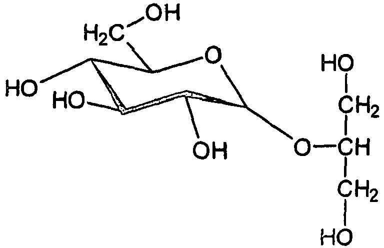 und/oder der allgemeinen Formel 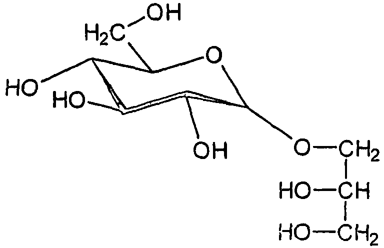
(ii) mindestens ein polymeres Verdickungsmittel gewählt aus der Gruppe der Acrylamidomethylpropylsulfonsäure-Polymere, dadurch gekennzeichnet, daß als Acrylamidomethylpropylsulfonsäure-Polymer das Ammoniumacryloyldimethyltaurat / VP Copolymer gewählt wird,
die Nachteile des Standes der Technik beseitigen.

Ferner war überraschend, daß die Verwendung von Acrylamidomethylpropylsulfonsäure-Polymeren zur erhöhung der Stabilität kosmetischer Zubereitungen den Nachteilen des Standes der Technik abhelfen würde.

Es war für den Fachmann ferner nicht vorauszusehen gewesen, daß die erfindungsgemäßen Wirkstoffkombinationen bzw. kosmetische oder dermatologische Zubereitungen, diese enthaltend
- besser als feuchtigkeitsspendendes Agens wirken
- besser gegen Austrocknung durch Emulgatoren und Coemulgatoren schützt
- besser die Barriereleistung der Haut steigern
- besser die Haut vor exogenen Noxen schützt
- besser gegen die Symptome der sensiblen Haut schützt
- besser die Hautrauhigkeit reduziert
- besser gegen die Hautalterung wirken
als die Wirkstoffe, Wirkstoffkombinationen und Zubereitungen des Standes der Technik.

Besonders bevorzugtes Glucosylglycerid ist das (2-O-β-D-Glucopyranosyl)-sn-glycerin.

Die AMPS-Polymere können als 100 % aktiv substanz oder als Inversverdicker kommerziell verfügbar sein.

### Beispiele :

vernetzte AMPS-Copolymere, Puderform:
Aristoflex AVC (Ammonium Acryloyldimethyltaurate/VP Copolymer, Clariant) Kosmetische öder dermatologische Zubereitungen im Sinne der vorliegenden Erfindung enthalten - bezogen auf das Gesamtgewicht der Zubereitungen - vorteilhaft 0,05 bis 5 Gew.-%, insbesondere 0,1 bis 3 Gew.-% an einem oder mehreren Acrylamidomethylpropylsulfonsäure Polymeren.

Es ist erfindungsgemäß vorteilhaft, das molare Verhältnis von einem oder mehreren Glucosylglyceriden zu einem oder mehreren Acrylamidomethylpropylsulfonsäure-Polymeren aus dem Bereich von 100 : 1 bis 1 : 100 , bevorzugt 50 : 1 bis 1 : 50, insbesondere bevorzugt 20 : 1 bis 1 : 20 zu wählen.

Entsprechend der erfindungsgemäßen Verwendung sind die Zubereitungen besonders vorteilhaft dadurch gekennzeichnet, daß das oder die Glucosylglyceride in Konzentrationen von 0,01 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegen.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wässrige Lösungen;
- Öle, wie Triglyceride der Caprin- oder der Caprylsäure, Fettsäureether wie Dicaprylylether, Kohlensäureester wie Dicaprylylcarbonat, sowie pflanzliche Triglyceride wie Sonnenblumenöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether, Ethylhexylglycerin, Methylpropandiol und analoge Produkte.

Insbesondere werden Gemische der vorstehend genannten Lösungsmittel verwendet. Bei alkoholischen Lösungsmitteln kann Wasser ein weiterer Bestandteil sein.

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische und / oder anorganische Verdickungsmittel enthalten. Weiterhin sind diese Verdickungsmittel Bestandteil kosmetischer Emulsionen.

Vorteilhafte Verdickungsmittel für derartige Zubereitungen sind beispielsweise Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon).

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 980, 981, 984 1342, 1382, 2984 und 5984. ebenso die ETD-Typen 2001, 2020, 2050 und Carbopol Ultrez 10, PVM/MA Decadien Crosspolymer (Handelsname Stabileze 06), Polyglycerylmethacrylat sowie Polyacrylamid. Weitere vorteilhafte Gelbildner für derartige Zubereitungen sind Xanthan Gummi, Polyvinylpyrrolidon, Cellulose Derivate, insbesondere Celluloseether wie zum Beispiel Hydroxypropylmethylcellulose, Stärke und Stärkederivate, Hyaluronsäure Johannisbrotkernmehl, Siliciumdioxid und Aluminiumsilikate.

Das Verdickungsmittel ist in dem Gel, der Dispersion bzw. der Emulsion z.B. in einer Menge zwischen 0,01 und 5 Gew.-%, bevorzugt zwischen 0,1 und 2 Gew.-%, enthalten.

Es folgen vorteilhafte Ausführungsbeispiele der vorliegenden Erfindung.

### Beispiel 1: O/W-Emulsionen:

| | Gew.-% |
|---|---|
| Glycerystearatcitrat | 2 |
| Cetylstearylalkohol | 3 |
| Myristylmyristat | 3 |
| Caprylsäure/Caprinsäuretriglyceride | 4 |
| Octyldodecanol | 1 |
| Dimethicon | 2 |
| α-Glucosylrutin | 0,1 |
| Citronensäure, Natriumsalz | 0,1 |
| Glycerylglucosid | 5 |
| Ethanol denaturiert | 3 |
| Ammoniumacryloyldimethyltaurat / VP Copolymer | 0,3 |
| Glycerin | 10 |
| Füllstoffe (Distärkephosphat, SiO₂, Talkum, Aluminiumstearat) | 0,1 |
| Parfüm / Farbstoffe | q.s. |
| Wasser | Ad.100 |

### Beispiel 2: O/W-Emulsionen:

| | Gew-% |
|---|---|
| Glycerystearatcitrat | 1,5 |
| Wollwachsalkohol | 0,5 |
| Stearylalkohol | 1 |
| Cetylstearylalkohol | 1 |
| Hydrierte Cocosfettglyceride (Hydrogenated Coco Glycerides) | 2 |
| Butylene Glycol Dicaprylat / Dicaprat | 1 |
| Ethyl Hexyl Cocoate | 3 |
| Vaseline | 4 |
| Dicaprylylether | 1 |
| Ethylhexylmethoxycinnamat | 3 |
| Bis-Ethylhexyloxyphenol-methoxyphenyltriazine | 1 |
| Ubichinon (Q10) | 0,05 |
| Glycerylglucosid | 5 |
| Iminodisuccinat | 0,1 |
| Phenoxyethanol | 0,3 |
| p-Hydroxybenzoesäurealkylester (Paraben) | 0,5 |
| Diazolidinylharnstoff | 0,25 |
| lodopropynylbutylcarbamat | 0,1 |
| Ethanol denaturiert | 1 |
| Ammoniumacryloyldimethyltaurat / VP Copolymer | 0,7 |
| Glycerin | 8 |
| Füllstoffe (Distärkephosphat, SiO₂, Talkum, Aluminiumstearat) | 1 |
| Parfüm / Farbstoffe | q.s. |
| Wasser | Ad 100 |

### Beispiel 3: O/W-Emulsionen:

| | Gew.-% |
|---|---|
| Glycerystearatcitrat | 3 |
| Cetylalkohol | 3 |
| Sheabutter | 2 |
| C₁₂₋₁₅ Alkyl Benzoate | 3 |
| Caprylsäure/Caprinsäure Triglyceride | 4 |
| Paraffinum Liquidum | 1 |
| Cyclomethicon | 1 |
| Dicaprylylether | 4 |
| Ethylhexylmethoxycinnamat | 2 |
| Ethylhexyltriazon | 2 |
| Butylmethoxydibenzoylmethan | 1 |
| Taurin | 1,0 |
| Tocopherylacetat | 0.5 |
| Glycerylglucosid | 5 |
| Trinatrium EDTA | 0.2 |
| p-Hydroxybenzoesäurealkylester (Paraben) | 0,4 |
| 1,3-Dimethylol-5,5-dimethyl-hydantoin(DMDM Hydantoin) | 0,2 |
| Ethanol denaturiert | 2 |
| Ammoniumacryloyldimethyltaurat / VP Copolymer | 0,5 |
| Glycerin | 10 |
| Butylenglycol | 1 |
| Füllstoffe (Distärkephosphat, SiO₂, Talkum, Aluminiumstearat) | 0,2 |
| Parfüm / Farbstoffe | q.s. |
| Wasser | Ad 100 |

### Beispiel 4: O/W-Emulsionen:

| | Gew.-% |
|---|---|
| Glycerylstearat, selbstemulgierend | 2 |
| Wollwachsalkohol | 0,5 |
| Stearinsäure | 2 |
| Myristylalkohol | 2 |
| Stearylalkohol | 0,5 |
| Cetylstearylalkohol | 2 |
| C₁₂₋₁₅ Alkylbenzoate | 3 |
| Cyclomethicon | 3 |
| Dimethicon | 1 |
| TiO₂ | 1 |
| 2-Ethylhexyl-2-cyano-3-diphenylacrylat (Octocrylen) | 5 |
| Kreatin | 0.3 |
| Kreatinin | 0.1 |
| Ubichinon (Q10) | 0.05 |
| Phytinsäure | 0,1 |
| Glycerylglucosid | 5 |
| p-Hydroxybenzoesäurealkylester (Paraben) | 0,3 |
| Hexamidindiisethionat | 0,04 |
| lodopropynylbutylcarbamat | 0.05 |
| Ammoniumacryloyldimethyltaurat / VP Copolymer | 0,2 |
| Glycerin | 5 |
| Butylenglycol | 2 |
| Füllstoffe (Distärkephosphat, SiO₂, Talkum, Aluminiumstearat) | 0,5 |
| Parfüm / Farbstoffe | q.s. |
| Wasser | Ad 100 |

### Beispiel 5: O/W-Emulsionen:

| | Gew.-% |
|---|---|
| Glycerylstearat | 2 |
| Cetylphosphat | 0,5 |
| Wollwachsalkohol | 0,5 |
| Stearinsäure | 3 |
| Myristylalkohol | 3 |
| Myristylmyristat | 1,5 |
| Hydrierte Cocosfettglyceride (Hydrogenated Coco Glycerides) | 1 |
| Vaseline | 2 |
| Cyclomethicon | 1 |
| Polydecene | 1 |
| TiO₂ | 1 |
| Ethylhexylmethoxycinnamat | 5 |
| Butylmethoxydibenzoylmethan | 1 |
| Natrium-Ascorbylphosphat | 0,2 |
| Glycerylglucosid | 5 |
| Trinatrium EDTA | 0,2 |
| Phenoxyethanol | 0,8 |
| p-Hydroxybenzoesäurealkylester (Paraben) | 0,4 |
| Hexamidindiisethionat | 0,05 |
| 2-Ethylhexylglycerinether (Octoxyglycerin) | 3 |
| Ammoniumacryloyldimethyltaurat / VP Copolymer | 0,1 |
| Glycerin | 15 |
| Füllstoffe (Distärkephosphat, SiO₂, Talkum, Aluminiumstearat) | 0,05 |
| Parfüm / Farbstoffe | q.s. |
| Wasser | Ad 100 |

### Beispiel 6: O/W-Emulsionen:

| | Gew.-% |
|---|---|
| PEG-40-Stearate | 2 |
| Glycerylstearat | 2 |
| Stearylalkohol | 0,5 |
| Cetylalkohol | 2 |
| C₁₂₋₁₅ Alkylbenzoate | 2 |
| Caprylsäure/Caprinsäure Triglyceride | 1 |
| Cyclomethicon | 3 |
| Dicaprylylcarbonat | 2 |
| Ethylhexylmethoxycinnamat | 3 |
| Ethylhexyltriazon | 2 |
| Bis-Ethylhexyloxyphenol-methoxyphenyltriazine | 1 |
| Ubichinon (Q10) | 0,02 |
| Creatin | 0,5 |
| Kreatinin | 0,1 |
| Weinsäure, Natriumsalz | 0.1 |
| Glycerylglucosid | 5 |
| Phenoxyethanol | 0,4 |
| Diazolidinylhanrstoff | 0,2 |
| Ethanol denaturiert | 8 |
| Ammoniumacryloyldimethyltaurat / VP Copolymer | 0,8 |
| Glycerin | 5 |
| Füllstoffe (Distärkephösphat, SiO₂, Talkum, Aluminiumstearat) | 0,1 |
| Parfüm / Farbstoffe | q.s. |
| Wasser | Ad 100 |

### Beispiel 7: Hydrodispersion / Gelcreme

| | Gew.-% |
|---|---|
| Cyclomethicon | 9,00 |
| Glycerylglucosid | 5 |
| Phenoxyethanol + Ethyl-, Methyl-, Propyl- Butyl-, Isobutylparaben | 0,50 |
| Parfum / Farbstoffe | q.s |
| Ammonium AcryloyldimethyltauratVP Copolymer | 1,0 |
| Glycerin | 10,00 |
| Wasser | ad 100,0 |

### Beispiel 8: Hydrodispersion / Gelcreme

| | Gew.-% |
|---|---|
| Dicaprylylcarbonat | 9,00 |
| Cyclomethicon | 2,00 |
| Glycerylglucosid | 5 |
| Phenoxyethanol + Ethyl-, Methyl-, Propyl- Butyl-, Isobutylparaben | 0,50 |
| Parfum / Farbstoffe | q.s |
| Ammonium Acryloyldimethyltaurat/VP Copolymer | 0,8 |
| Glycerin | 10,00 |
| Wasser | ad 100,0 |

### Beispiel 9: Hydrodispersion / Gelcreme

| | Gew.-% |
|---|---|
| Caprytsäure/Caprinsäuretriglycerid | 4,00 |
| Cyclomethicon | 3,00 |
| Glycerylglucosid | 5 |
| Phenoxyethanol + Ethyl-, Methyl-, Propyl- Butyl-, Isobutylparaben | 0,50 |
| Parfum / Farbstoffe | q.s |
| Ammonium AcryloyldimethyltauratNP Copolymer | 0,5 |
| Glycerin | 10,00 |
| Wasser | ad 100,0 |

### Beispiel 10: Hydrodispersion / Gelcreme

| | Gew.-% |
|---|---|
| Shea Butter | 1,00 |
| Octyldodecanol | 1,50 |
| Dicaprylylcarbonat | 1,00 |
| Dimethicon | 0,50 |
| Cyclomethicon | 2,00 |
| Glycerylglucosid | 5 |
| Phenoxyethanol + Ethyl-, Methyl-, Propyl- Butyl-, Isobutylparaben | 0,50 |
| Parfum / Farbstoffe | q.s |
| Ammonium Acryloyldimethyltaurat/VP Copolymer | 0,8 |
| Alkohol Denat | 3,00 |
| Glycerin | 10,00 |
| Wasser | ad 100,0 |

### Beispiel 11: Hydrodispersion / Gelcreme

| | Gew.-% |
|---|---|
| Mineralöl | 6,00 |
| Dicaprylylcarbonat | 3,00 |
| Cyclomethicon | 1,00 |
| Glycerylglucosid | 5 |
| Phenoxyethanol + Ethyl-, Methyl-, Propyl- Butyl-, Isobutylparaben | 0,50 |
| Parfum / Farbstoffe | q.s |
| Ammonium Acryloyldimethyltaurat/VP Copolymer | 1,0 |
| Glycerin | 10,00 |
| Wasser | ad 100,0 |

## Patentansprüche

1. Kosmetische Zubereitungen, enthaltend
(i) ein oder mehrere Glucosylglyceride gewählt aus den Substanzen
der allgemeinen Formel 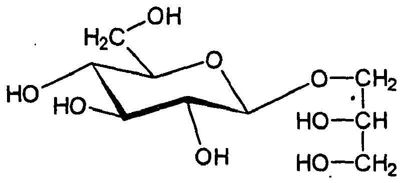 und/oder der allgemeinen Formel 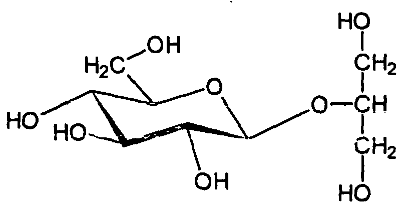 und/oder der allgemeinen Formel 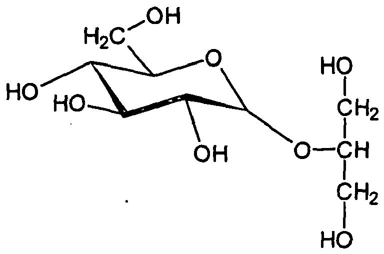 und/oder der allgemeinen Formel 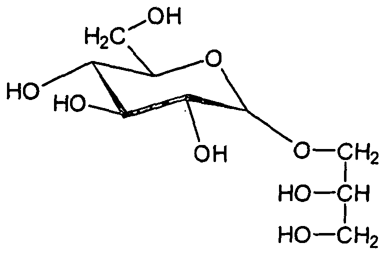
(ii) mindestens ein polymeres Verdickungsmittel gewählt aus der Gruppe der Acrylamidomethylpropylsulfonsäure-Polymere,
**dadurch gekennzeichnet, daß** als Acrylamidomethylpropylsulfonsäure-Polymer das Ammoniumacryloyldimethyltaurat / VP Copolymer gewählt wird.

2. Zubereitungen gemäß Anspruch 1, in denen Glucosylglycerid in Konzentrationen von 0,01 - 10,00 Gew.%, bevorzugt 0,05 - 5,00 Gew.-%, besonders bevorzugt 0,1 - 3,00 Gew.-%, jeweils bezogen auf das Gesamtgewicht der Zusammensetzung, vorliegt.

3. Zubereitungen gemäß Anspruch 1, in denen das oder die hydrophobisierten Acrylamidomethylpropylsulfonsäure-Polymere in Konzentrationen von 0,01 - 10,00 Gew.-%, bevorzugt 0,05 - 5,00 Gew.-%, besonders bevorzugt 0,1 - 3,00 Ges.-%, jeweils bezogen auf das Gesamtgewicht der Zusammensetzung, vorliegt oder vorliegen

## Claims

1. Cosmetic preparations comprising
(i) one or more glucosyl glycerides selected from the substances of the general formula 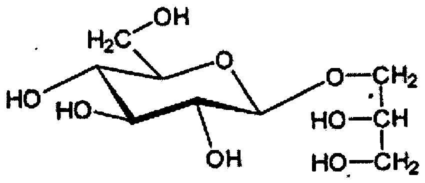 and/or of the general formula 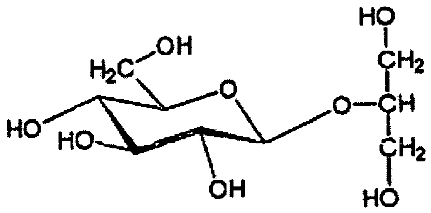 and/or of the general formula 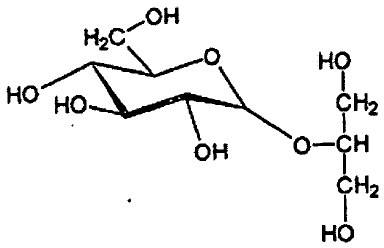 and/or of the general formula 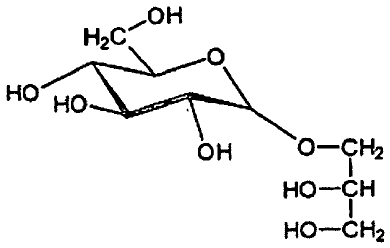
(ii) at least one polymeric thickener selected from the group of the acrylamidomethylpropylsulphonic acid polymers, **characterized in that** the ammonium acryloyldimethyltaurate/VP copolymer is selected as acrylamidomethylpropylsulphonic acid polymer.

2. Preparations according to Claim 1, in which glucosyl glyceride is present in concentrations of 0.01-10.00% by weight, preferably 0.05-5.00% by weight, particularly preferably 0.1-3.00% by weight, in each case based on the total weight of the composition.

3. Preparations according to Claim 1, in which the hydrophobicized acrylamidomethylpropylsulphonic acid polymer or polymers is or are present in concentrations of 0.01-10.00% by weight, preferably 0.05-5.00% by weight, particularly preferably 0.1-3.00% by weight, in each case based on the total weight of the composition.

## Revendications

1. Préparations cosmétiques, contenant
(i) un ou plusieurs glucosylglycérides choisis parmi les substances
de formule générale 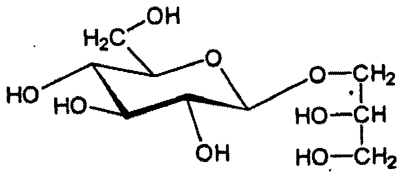 et/ou de formule générale 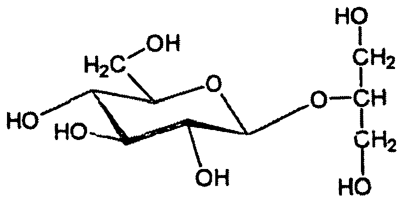 et/ou de formule générale 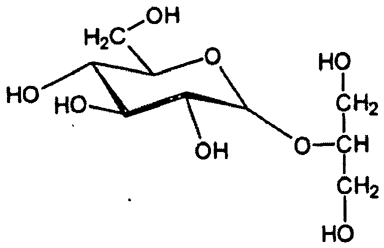 et/ou de formule générale 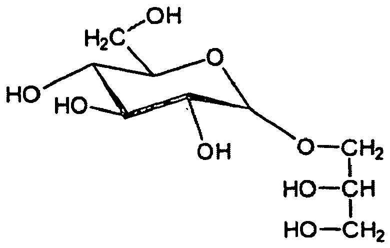
(ii) au moins un épaississant polymère choisi dans le groupe des polymères de l'acide acrylamidométhylpropylsulfonique,
**caractérisées en ce que** le copolymère d'acryloyldiméthyltaurate d'ammonium/VP est choisi en tant que polymère de l'acide acrylamidométhylpropylsulfonique.

2. Préparations selon la revendication 1, dans lesquelles le glucosylglycéride est présent en concentrations de 0,01 à 10,00 % en poids, de préférence de 0,05 à 5,00 % en poids, de manière particulièrement préférée de 0,1 à 3,00 % en poids, à chaque fois par rapport au poids total de la composition.

3. Préparations selon la revendication 1, dans lesquelles le ou les polymères de l'acide acrylamidométhylpropylsulfonique hydrophobés sont présents en concentrations de 0,01 à 10,00 % en poids, de préférence de 0,05 à 5,00 % en poids, de manière particulièrement préférée de 0,1 à 3,00 % en poids, à chaque fois par rapport au poids total de la composition.
